# EUROPÄISCHE PATENTSCHRIFT

(11) **EP 1 656 071 B1**
(45) Veröffentlichungstag und Bekanntmachung des Hinweises auf die Patenterteilung: **30.04.2008**
(21) Anmeldenummer: 04718355.3
(22) Anmeldetag: 08.03.2004
(51) Int. Cl.: A61B 17/17

(54) **GEBOGENES POSITIONIER- UND EINFÜHRWERKZEUG FÜR DAS ANBRINGEN EINES FÜHRUNGSDRAHTES IN DEN FEMUR**
CURVED POSITIONING AND INSERTION INSTRUMENT FOR INSERTING A GUIDE WIRE INTO THE FEMUR
INSTRUMENT COURBE DE POSITIONNEMENT ET D'INTRODUCTION D'UN FIL METALLIQUE DE GUIDAGE DANS LE FEMUR

(30) Priorität: 13.08.2003 CH 139503; 23.12.2003 CH 26042003
(43) Veröffentlichungstag der Anmeldung: 17.05.2006
(73) Patentinhaber: Synthes GmbH, 4436 Oberdorf (CH)
(72) Erfinder: KAUP, Thomas, 6003 Luzern (CH); FERNANDEZ DELL'OCA, Alberto, Uruguay-Montevideo (UY)
(74) Vertreter: Rosenich, Paul
(86) Internationale Anmeldenummer: PCT/IB2004/000624
(87) Internationale Veröffentlichungsnummer: WO 2005/016155

(56) Entgegenhaltungen:
- US-A- 5 207 753
- US-A- 6 074 392
- US-B1- 6 309 396
- PATENT ABSTRACTS OF JAPAN vol. 2000, no. 26, 1. Juli 2002 (2002-07-01) & JP 2001 245894 A (SAKAMOTO MASASHI), 11. September 2001 (2001-09-11)

## Beschreibung

Die Erfindung betrifft ein gebogenes Positionier- und Einführwerkzeug für das Anbringen eines Führungsdrahtes in den Femur zur präzisen Knocheneröffnung.

Eine präzise Knocheneröffnung bedingt eine verlässliche Referenz in Position und Richtung an der sich ein Operateur mit einem Bohrer bzw. Fräser zu leiten vermag. Es ist bekannt, dass zu diesem Zwecke Führungsdrähte verwendet werden, die vor der Bohrung in den Femur eingeführt (eingebohrt oder eingeschlagen) werden, um den Bohrer sicher zu führen.

Die US-A-6074392 (Basis für den Oberbegriff des Anspruchs 1) beschreibt ein solches gebogenes Positionier- und Einführwerkzeug für das Anbringen eines Führungsdrahtes in den Femur mit einem gebogenen Führungsrohr mit einem distalen Ende zum Ansetzen am Trochanter und einem proximalen Ende für das Einschieben eines Führungsdrahtes, mit einem Haltearm für die Verbindung zwischen dem Führungsrohr und einem Handgriff. Dieser Aufbau ist so gewählt worden, weil das Einführen eines Führunsgdrahtes entlang einer gekrümmten Kurve hinsichtlich der Anatomie der menschlichen Femora vorteilhaft ist, und deshalb das Gewebe im betroffenen Bereich bei einer Operation weniger beeinträchtigt wird.

Die US-A-6074392 beschreibt also einen Werkzeugaufbau mit einem Schlüsselelement, das aus einem gekrümmten Führungsrohr für einen gekrümmten Führungsdraht besteht. Dieser Führungsdraht wird in den Knochen eingeführt und dient als Führungspfad für einen hohlen und flexiblen Bohrer. Der Führungsdraht wird mit Hilfe eines Positionier- und Einführwerkzeugs montiert, wobei das gebogene Führungsrohr, mittels eines Trägerteils an einem Griff angebracht ist, der vom Operateur gut gehalten werden kann. Die genaue Positionierung des Führungsrohres wird durch Röntgenstrahlen oder durch direkten Augenschein vollzogen. Nach der Positionierung wird der Führungsdraht entlang der Längsachse des Knochens hineingehämmert oder auf eine andere Weise hineingetrieben. Danach wird das Einführwerkzeug entfernt und der flexible Bohrer bzw. Fräser über den Führungsdraht geführt. Nach vollzogener Bohrung wird alles entfernt.

Der Schwachpunkt dieser Lösung zeigt sich aber darin, dass sich keine besondere Korrekturmöglichkeit bietet, sollte sich auf dem Röntgenbild zeigen, dass der Führungsdraht nicht optimal zu liegen gekommen ist.

In einem völlig anderen Aufbau z.B. gemäss US-A-5951561 wurde ebenso ein Führungswerkzeug angegeben, das über die Möglichkeit der Korrektursetzung von Führungsdrähten verfügt. Dieser Aufbau besteht jedoch aus einer voluminösen Ummantelung, die ein rotatives zylindrisches Bauteil aus mehreren Scheiben mit je mehreren Löchern aufnimmt, die in einem aufwändigen Herstellverfahren konzentrisch zueinander ausgerichtet werden müssen und zudem keine Möglichkeit bieten, einen Führungsdraht entlang einer gekrümmten Kurve in den Femur einzuschlagen. Insofern bietet dieser Aufbau keine Verbesserung für einen gattungsgemäßen Aufbau an.

Der Erfindung liegt nun die Aufgabe zugrunde, mit einem einfachen Gerät Korrektursetzungen zu ermöglichen, wobei das Gewebe im betroffenen Bereich so wenig als möglich beeinträchtigt werden soll. Aus diesem Grunde hätte der Fachmann, wie schon oben angemerkt, die US-A-5951561 gar nicht als Stand der Technik für die Lösung der vorliegenden Aufgabe herangezogen. Auch ist nachvollziehbar, dass die im Jahre 1998 veröffentlichte US-A-6074392 bereits eine Weiterentwicklung der im Jahre 1997 veröffentlichten US-A-5624447 ist, während die US-A-5951561 im selben Jahr wie die US-A-6074392 veröffentlicht wurde, und also parallel einen anderen Weg zum Setzen von Führungsdrähten verfolgte.

Weitere, jedoch weniger relevante, Dokumente zum Stand der Technik sind die US-A-4466429, US-A-3439671, US-A-5135527, US-A-5112336, US-A-4712541, US-B1-6273892.

Der Erfinder stellt nun zur Lösung der Aufgabe ein neues Positionier- und Einführwerkzeug vor, das einen Handgriff umfasst, der mittels eines Haltearms an einem gebogenen Führungsrohr angeordnet ist. An dessen Ende befindet sich ein Positionierhaken mit gegebenenfalls einer Führungsbohrung, die bezüglich des Führungsrohrs in einem Winkel von 6°-30°, vorzugsweise 7°-20°, insbesondere ca. 8° angestellt ist. Neben dieser Bohrung sind gemäss einer verbesserten Weiterentwicklung verschiedene Korrekturbohrungen vorhanden. Des Weiteren wird bei der Anwendung des erfindungsgemäßen Werkzeugs ein T-Griff benötigt. Mit der Erfindung kann man somit mindestens einen Führungsdraht einsetzen, der später einen Öffnungsbohrer führt. Anstelle eines T-Griffs oder eines motorisierten Bohr- oder Schlagantriebes kann auch ein Schlagkopf vorgesehen sein, so dass der Führungsdraht eingehämmert werden kann.

Bei der praktischen Anwendung wird zunächst das Führungsrohr gegebenenfalls mit einem Referenz-Führungsdraht geladen. Der Handgriff des Positionier- und Einführwerkzeuges wird dann nach Augenmass etwa parallel zur Längsachse des Femur und auf die Verlängerung dessen intramedulären Kanals ausgerichtet. Der Positionierhaken wird auf den Trochanter platziert und die Nase des Hakens durch Druck nach medial in den Muskel (gluteus medius) gedrückt, wobei der Referenz-Führungsdraht parallel zur Längsachse des Femur eingeführt wird. Das Werkzeug wird entfernt. Dann wird das Führungsrohr des Positionier- und Einführwerkzeugs mit einem Öffnungs- Führungsdraht geladen und gemäss dieser Anwendungsmethode die Führungsbohrung im Positionierhaken über den Referenz-Führungsdraht geschoben und somit erneut auf dem Trochanter platziert. Weil die Führungsbohrung im Positionierhaken etwa 8° angestellt ist, richtet sich das Positionier- und Einführwerkzeug dementsprechend aus, um dem Öffnungs- Führungsdraht den richtigen Winkel zu geben. Diese Prozedur wird durchgeführt, weil ein bestimmter Winkel von Auge schlechter abzuschätzen ist, als eine Parallelität. Wenn nun aber bereits der Referenz-Führungsdraht zufällig auf Anhieb passen würde, dann könnte auch dieser als Öffnungs-Führungsdraht genutzt werden. In der Regel wird jedoch erwartet, dass ein zweiter, der Öffnungs- Führungsdraht gesetzt werden muss.

Eine Reihe von Ausgestaltungsvarianten ohne Führungsbohrung verzichtet auf die Einführung des Referenz-Führungsdrahtes und gewährleistet die Platzierung des Positionier- und Einführwerkzeuges am richtigen Ort und Winkel durch die den anatomischen Verhältnissen angepasste besondere Beschaffenheit ihrer Positionierhaken. Die Ausgestaltung dieser Haken ist also so gewählt, dass der Haken beim leichten Eindrücken in den medialen Muskel und beim Aufsetzen auf den Trochanter in der Mehrzahl der Anwendungen in der optimalen Position für das Einbringen des Öffnungsführungsdrahtes zu liegen kommt. Die Nase ist so gestaltet, dass sie weichteilschonend in den medialen Muskel gedrückt werden kann. Ihre Dicke liegt bei ca. 0.5-10mm, vorzugsweise ca. 1-5mm und insbesondere ca. 2.5mm. Die Länge zwischen dem Zentrum des Führungsrohres und der Nasenspitze des Positionierhakens kann entsprechend der Weichteilsituation variieren zwischen ca. 10-30mm, vorzugsweise ca. 15-25mm und beträgt insbesondere ca. 20mm. Die Korrekturbohrungen haben einen Durchmesser von ca. 1-5mm, vorzugsweise ca. 1.8-3.6 und insbesondere ca. 3.3mm. Die Aufnahme für das Führungsrohr ist ca. 3-20mm, vorzugsweise ca. 6-10mm und insbesondere ca. 8mm.

Wenn auch der Öffnungs- Führungsdraht in den Trochanter eingeführt ist, befindet er sich in der Frontalebene an seiner Einführstelle etwa in einem optimalen Winkel von etwa 8° zum Referenz-Führungsdraht. Röntgenbilder können nun bestätigen, ob der Öffnungs- Führungsdraht in der korrekten Position sitzt, um die Öffnung zu vollziehen. Wenn dieser nun aber beispielsweise zu nahe am lateralen Kortex sitzt, so besteht die Möglichkeit einer Korrektur mit Hilfe des Positionier- und Einführwerkzeugs. Dieses wird dann mit einer seiner Korrekturbohrungen über den Referenz- oder Öffnungs-Führungsdraht geschoben und ein weiterer, vorher schon geladener Öffnungs-Führungsdraht in neuer Position in den Trochanter eingeführt. Der alte Öffnungs-Führungsdraht, der nicht korrekt positioniert war, kann dann entfernt werden. Bei Bedarf kann eine solche Korrekturmaßnahme bereits anhand des einzig gesetzten Referenz-Führungsdrahtes dadurch getroffen werden, dass anstelle der Führungsbohrung gleich eine Korrekturbohrung auf den Referenz-Führungsdraht geschoben wird.

Weitere Ausbildungen der Erfindung sind in den Figuren und in den abhängigen Patentansprüchen angegeben.

Die Bezugszeichenliste ist Bestandteil der Offenbarung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei schematisch
Fig.1 - das Positionier- und Einführwerkzeug mit einem T-Griff, Führungsdrähten und einem Öffnungsbohrer;
Fig.2 - das Positionier- und Einführwerkzeug mit geladenem Führungsdraht;
Fig.3a - den Positionierhaken des Positionier- und Einführwerkzeuges mit seinen Führungs- und Korrekturbohrungen und der Nase für den Halt im medialen Muskel in frontal Ansicht bzw. anterior/ posterior Ansicht;
Fig.3b - den Positionierhaken des Positionier- und Einführwerkzeuges mit seinen Führungs- und Korrekturbohrungen und der Nase für den Halt im medialen Muskel in Ansicht A gemäss Fig.3a;
Fig.4 - das auf den Trochanter platzierte Positionier- und Einführwerkzeug mit Führungsdraht und T-Griff in Bohrbereitschaft in frontal Ansicht bzw. anterior/ posterior Ansicht;
Fig.5 - das mit dem Öffnungs-Führungsdraht geladene und über den Referenz-Führungsdraht geschobene Positionier- und Einführwerkzeug;
Fig.6 - das mit dem Offnungs-Führungsdraht geladene, über den Referenz-Führungsdraht gefahrene und auf den Trochanter erneut platzierte Positionier- und Einführwerkzeug mit T-Griff in Bohrbereitschaft;
Fig.7 - den über den Offnungs-Führungsdraht geschobene Öffnungsbohrer und T-Griff in Bohrbereitschaft;
Fig.8 - ein Röntgenbild des Femur mit eingeführtem Führungsdraht;
Fig.9 - ein Röntgenbild des Femur mit eingeführtem Referenz-Führungsdraht und einzuführendem Offnungs-Führungsdraht;
Fig. 10 - ein Röntgenbild des Femur mit eingeführtem Referenz-Führungsdraht und eingeführtem Offnungs-Führungsdraht;
Fig.11 - ein Röntgenbild des Femur mit eingeführtem Öffnungs-Führungsdraht und ohne, oder gegebenenfalls mit entferntem Referenz-Führungsdraht;
Fig.12 - ein Röntgenbild des Femur mit nicht optimal positioniertem ersten Öffnungs-Führungsdraht und zweitem Offnungs-Führungsdraht zur Korrektur;
Fig.13 - ein Röntgenbild des Femur mit korrigiert gesetztem Offnungs-Führungsdraht und
Fig.14 - ein Röntgenbild des Femur mit Öffnungsbohrung.
Fig.15a - den Positionierhaken des Positionier- und Einführwerkzeuges ohne Führungsbohrung und mit seinen Korrekturbohrungen und der Nase für den Halt im medialen Muskel in frontal Ansicht bzw. anterior/ posterior Ansicht;
Fig.15b - den Positionierhaken des Positionier- und Einführwerkzeuges ohne Führungsbohrung und mit seinen Korrekturbohrungen und der Nase für den Halt im medialen Muskel in Ansicht A gemäss Fig.15a;
Fig.16a - den Positionierhaken des Positionier- und Einführwerkzeuges ohne Führungsbohrung und mit seinen Korrekturbohrungen und der Nase für den Halt im medialen Muskel in frontal Ansicht bzw. anterior/ posterior Ansicht;
Fig.16b - den Positionierhaken des Positionier- und Einführwerkzeuges ohne Führungsbohrung und mit seinen Korrekturbohrungen und der Nase für den Halt im medialen Muskel in Ansicht A gemäss Fig.16a;
Fig.17a - den Positionierhaken des Positionier- und Einführwerkzeuges ohne Führungsbohrung und mit seinen Korrekturbohrungen und der Nase für den Halt im medialen Muskel in frontal Ansicht bzw. anterior/ posterior Ansicht;
Fig.17b - den Positionierhaken des Positionier- und Einführwerkzeuges ohne Führungsbohrung und mit seinen Korrekturbohrungen und der Nase für den Halt im medialen Muskel in Ansicht A gemäss Fig.17a;
Fig.18a - den Positionierhaken des Positionier- und Einführwerkzeuges ohne Führungsbohrung und mit seinen Korrekturbohrungen und der Nase für den Halt im medialen Muskel in frontal Ansicht bzw. anterior/ posterior Ansicht;
Fig.18b - den Positionierhaken des Positionier- und Einführwerkzeuges ohne Führungsbohrung und mit seinen Korrekturbohrungen und der Nase für den Halt im medialen Muskel in Ansicht A gemäss Fig.18a;
Fig.19a - den Positionierhaken des Positionier- und Einführwerkzeuges ohne Führungsbohrung und mit seinen (Zusatz-)Korrekturbohrungen und der Nase für den Halt im medialen Muskel in frontal Ansicht bzw. anterior/posterior Ansicht;
Fig.19b - den Positionierhaken des Positionier- und Einführwerkzeuges ohne Führungsbohrung und mit seinen (Zusatz-)Korrekturbohrungen und der Nase für den Halt im medialen Muskel in Ansicht A gemäss Fig.15a;

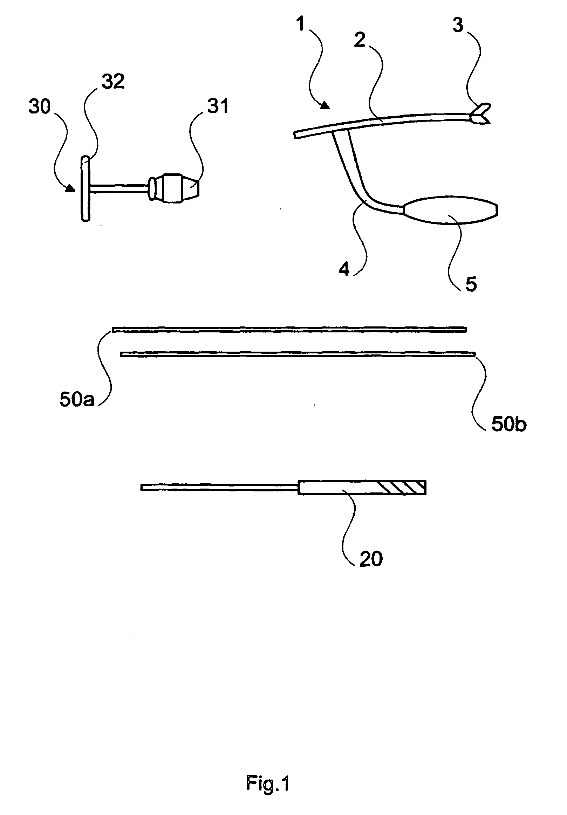

Fig.1 stellt die Einzelkomponenten dar, die bei der Anwendung des erfindungsgemäßen Werkzeuges in der Regel benötigt werden. Das Positionier- und Einführwerkzeug 1 besteht aus einem gekrümmten Führungsrohr 2, an dessen distalem Ende ein Positionierhaken 3 angeordnet ist. Die Krümmung des Führungsrohres 2 beschreibt einen Radius von ca. 300 bis ca. 800 mm, vorzugsweise 500 bis 700 mm, insbesondere ca. 600 mm. Der Positionierhaken 3 ist so ausgebildet, dass er auf der Spitze des großen Trochanters und dem medialen Muskel Halt findet. Im Bereich des proximalen Endes des Führungsrohres 2 ist ungefähr senkrecht dazu ein Haltearm 4 angebracht, der sich dann krümmt, um etwa parallel zum Führungsrohr 2 zu verlaufen. Am Ende des Haltearms 4 befindet sich ein Handgriff 5, dessen Längsachse etwa parallel zu einer Tangente auf das Führungsrohr 2 im Bereiche des Positionierhakens 3 ist.
Des Weiteren stellt Fig.1 zwei Führungsdrähte 50 dar, die so beschaffen sind, dass sie in das Führungsrohr 2 eingeführt, und je nach dem als Referenz-Führungsdraht 50a, respektive als Öffnungs-Führungsdraht 50b verwendet werden können. Zudem ist ein hohler Öffnungsbohrer 20 dargestellt, der so ausgebildet ist, dass er über einen Führungsdraht 50 geführt, sowie von einem T-Griff 30 betrieben werden kann.
Schließlich zeigt Fig.1 den eben erwähnten T-Griff 30 mit einem Hebel 32 und einem Bohrfutter, das sowohl auf die Führungsdrähte 50 als auch auf den Öffnungsbohrer 20 passt. Alternativ zum T-Griff sind natürlich auch motorisch angetriebene Bohr- oder Schlagantriebe möglich.

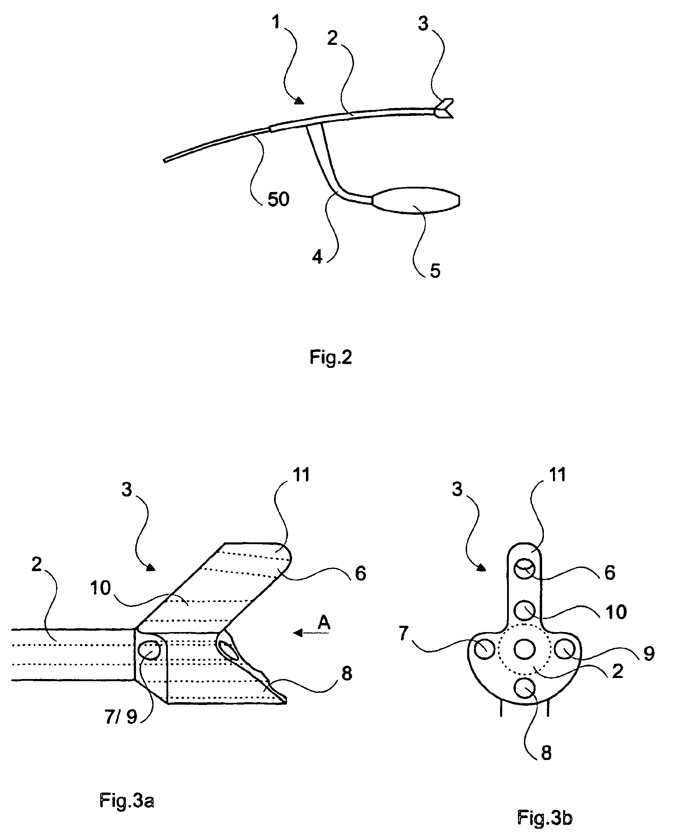

Fig.2 stellt das Positionier- und Einführwerkzeug 1 mit einem ins Führungsrohr 2 geladenen Führungsdraht 50 dar. Der Führungsdraht 50 kann am proximalen oder distalen Ende des Positionier- und Einführwerkzeugs 1 eingeführt werden, was zu einer zumindest vorübergehenden Krümmung des Führungsdrahtes 50 führt.

Fig.3a und Fig.3b stellen den Positionierhaken 3 des Positionier- und Einführwerkzeuges 1 dar, mit einer Führungsbohrung 6, die bezüglich des Führungsrohrs 2 und dessen zentraler Bohrung in einem Winkel von etwa 8° in der Nase 11 angestellt ist. Neben dieser Bohrung sind verschiedene Korrekturbohrungen vorhanden, insbesondere eine anterior/posterior Korrekturbohrung 7, eine mediale Korrekturbohrung 8, eine posterior/anterior Korrekturbohrung 9 und eine laterale Korrekturbohrung 10.

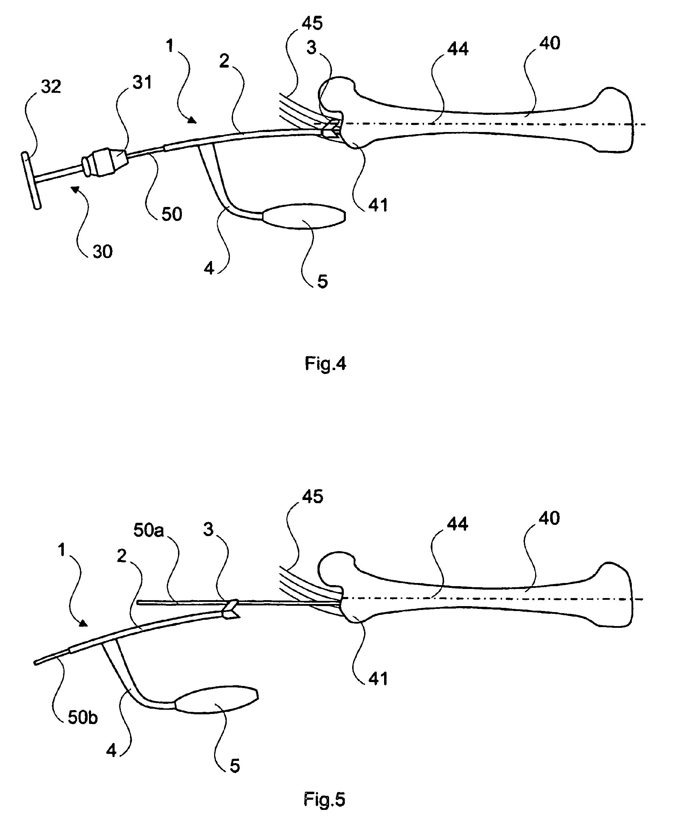

Fig.4 stellt das Positionier- und Einführwerkzeug 1 mit einem ins Führungsrohr 2 geladenen Führungsdraht 50 dar, das auf dem großen Trochanter 41 eines Femur 40 platziert ist. Die Beschaffenheit des Positionierhakens 3 gewährleistet eine saubere rutschsichere Positionierung auf der Oberfläche des Trochanter 41 und im medialen Muskel 45. Der Handgriff 5 des Positionier- und Einführwerkzeuges 1 ist parallel zur Längsachse des Femur 40 und parallel zu der Verlängerung dessen intramedulären Kanals ausgerichtet. Der T-Griff 30 ist in der Stellung gezeigt, wie er den Führungsdraht 50 einzuführen vermag.

Fig.5 stellt das Positionier- und Einführwerkzeug 1 dar, das gerade entlang seiner Führungsbohrung 6 auf den bereits in den Trochanter 41 des Femur 40 eingeführten Referenz-Führungsdraht 50a geschoben wird. Das Positionier- und Einführwerkzeug 1 bzw. sein Führungsrohr 2 ist mit einem Öffnungs-Führungsdraht 50b geladen.

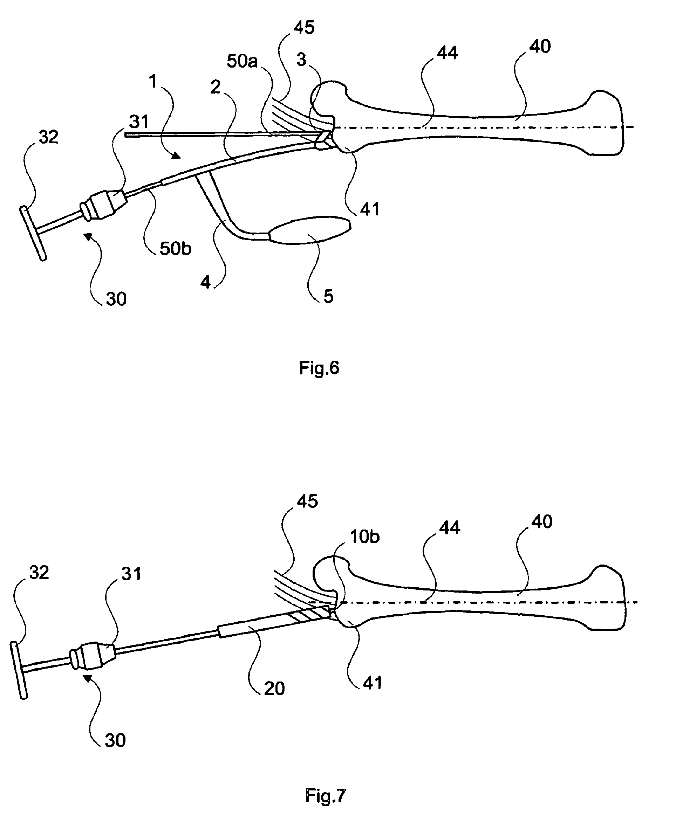

Fig.6 stellt das Positionier- und Einführwerkzeug 1 dar, das durch die Führungsbohrung 6 hindurch über den bereits in den Trochanter 41 des Femur 40 eingeführten Referenz-Führungsdraht 50a geschoben ist. Das Positionier- und Einführwerkzeug 1 ist entsprechend des Winkels von etwa 8° in der Frontalebene der Führungsbohrung 6 richtig positioniert, um den bereits geladenen Öffnungs-Führungsdraht 50b einzuführen. Der T-Griff 30 ist in der Stellung gezeigt, wie er den Öffnungs-Führungsdraht 50b einzuführen vermag.

Fig.7 stellt den Öffnungsbohrer 20 dar, wie er über den eingeführten Öffnungs-Führungsdraht 50b geschoben ist. Der T-Griff 30 ist in der Stellung gezeigt, wie er den Öffnungsbohrer 20 in den Trochanter 41 des Femur 40 einzuführen vermag.

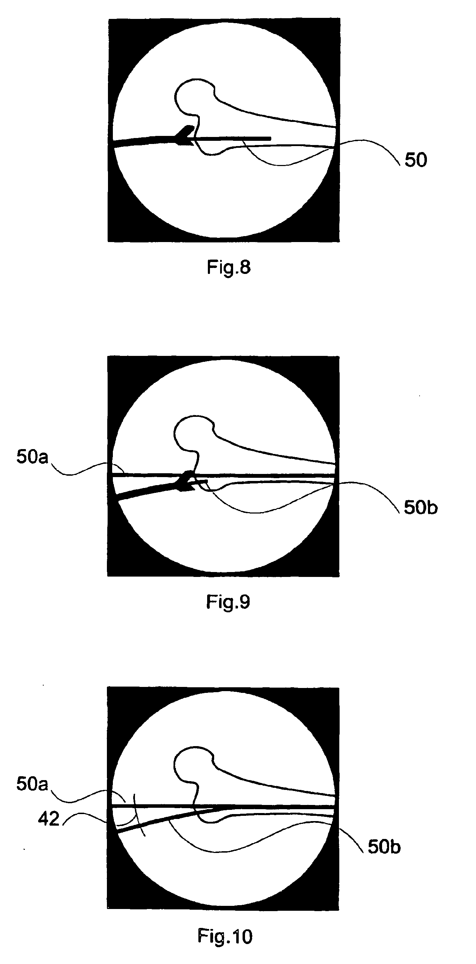

Fig.8 stellt ein Röntgenbild dar, welches den Positionierhaken 3 des Positionier- und Einführwerkzeugs 1 und einen im Führungsrohr 2 geladenen und in den Trochanter 41 des Femur 40 eingeführten Führungsdraht 50 zeigt. Es ist eine saubere Positionierung durch die Beschaffenheit des Positionierhakens 3 auf der Oberfläche des Trochanter 41 erkennbar. Der Führungsdraht 50 ist parallel zur Längsachse des Femur 40 und in dessen intramedulären Kanal eingeführt.

Fig.9 stellt ein Röntgenbild dar, welches den Positionierhaken 3 des Positionier- und Einführwerkzeugs 1 zeigt, der über seine Führungsbohrung 6 über den bereits in den Trochanter 41 des Femur 40 eingeführten Referenz-Führungsdraht 50a geschoben ist. Das Positionier- und Einführwerkzeug 1 ist entsprechend des Winkels von etwa 8° in der Frontalebene der Führungsbohrung 6 optimal richtig positioniert, um den bereits geladenen Öffnungs-Führungsdraht 50b einzuführen.

Fig.10 stellt ein Röntgenbild dar, welches den in den Trochanter 41 des Femur 40 eingeführten Referenz-Führungsdraht 50a und Öffnungs-Führungsdraht 50b zeigt. Der angestrebte Einführungswinkel 42 von etwa 8° zwischen Referenz-Führungsdraht 50a und Öffnungs-Führungsdraht 50b ist gut erkennbar.

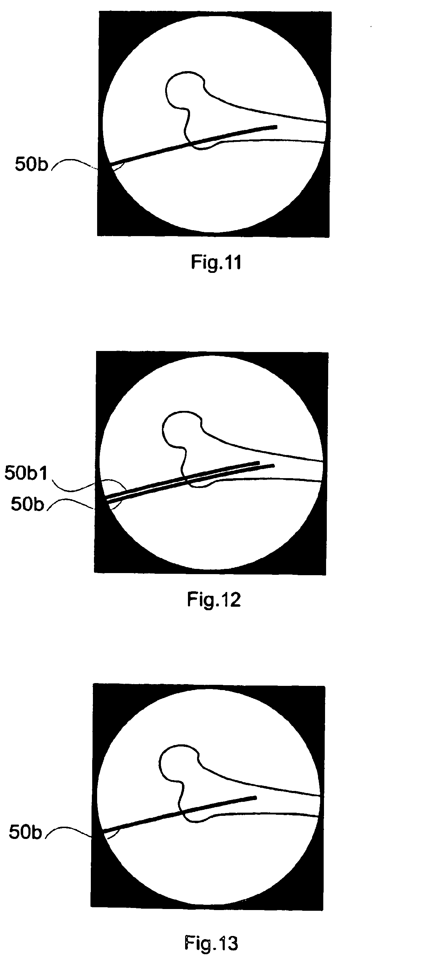

Fig.11 stellt ein Röntgenbild dar, welches den verbleibenden Öffnungs-Führungsdraht 50b gegebenenfalls nach dem Entfernen des Referenz-Führungsdrahtes 50a zeigt. Der Öffnungs-Führungsdraht 50b sitzt in diesem Beispiel zu nahe am lateralen Kortex um die Öffnung zu vollziehen und muss korrigiert werden.

Fig.12 stellt ein Röntgenbild dar, welches die Korrektur des Öffnungs-Führungsdrahtes 50b zeigt. Ein zweiter Öffnungs-Führungsdraht 50b ist parallel zum Führungsdraht 50b in den Trochanter 41 eingeführt.

Fig.13 stellt ein Röntgenbild dar, welches den korrigierten öffnungs-Führungsdraht 50b nach der Entfernung des ersten Öffnungs-Führungsdrahtes 50b zeigt. Er liegt nun optimal, um die Öffnung mittels Öffnungsbohrer 20 zu vollziehen.

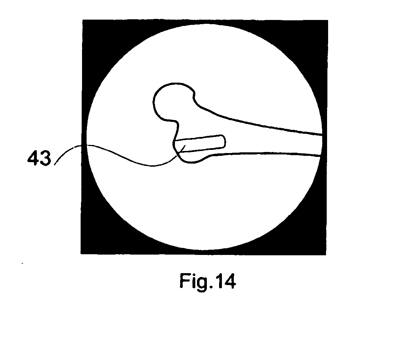

Fig.14 stellt ein Röntgenbild dar, welches die vollzogene Öffnung 43 des Trochanter 41 des Femur 40 zeigt.

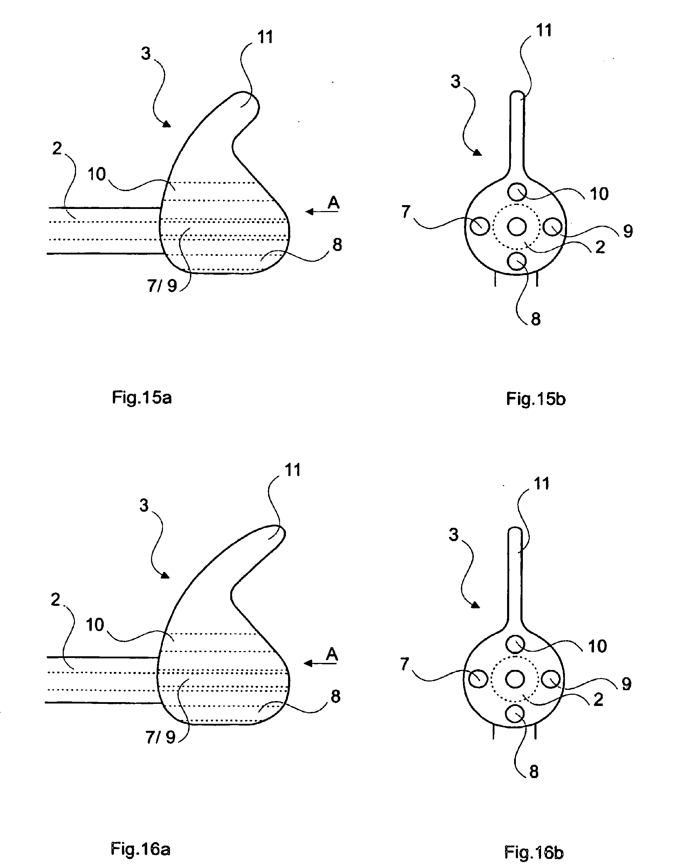

Fig.15a und Fig.15b stellen den Positionierhaken 3 des Positionier- und Einführwerkzeuges 1, der keine Führungsbohrung aufweist dar, mit verschiedenen Korrekturbohrungen, insbesondere eine anterior/posterior Korrekturbohrung 7, eine mediale Korrekturbohrung 8, eine posterior/anterior Korrekturbohrung 9 und eine laterale Korrekturbohrung 10. Im Vergleich zu der in Fig.3a und Fig.3b dargestellten Variante ist zudem die Nase 11 markant dünner.

Fig.16a und Fig.16b stellen den Positionierhaken 3 des Positionier- und Einführwerkzeuges 1, der keine Führungsbohrung aufweist dar, mit verschiedenen Korrekturbohrungen, insbesondere eine anterior/posterior Korrekturbohrung 7, eine mediale Korrekturbohrung 8, eine posterior/anterior Korrekturbohrung 9 und eine laterale Korrekturbohrung 10. Im Vergleich zu der in Fig.15a und Fig.15b dargestellten Variante läuft die Nase 11 etwas spitzer aus und ist länger.

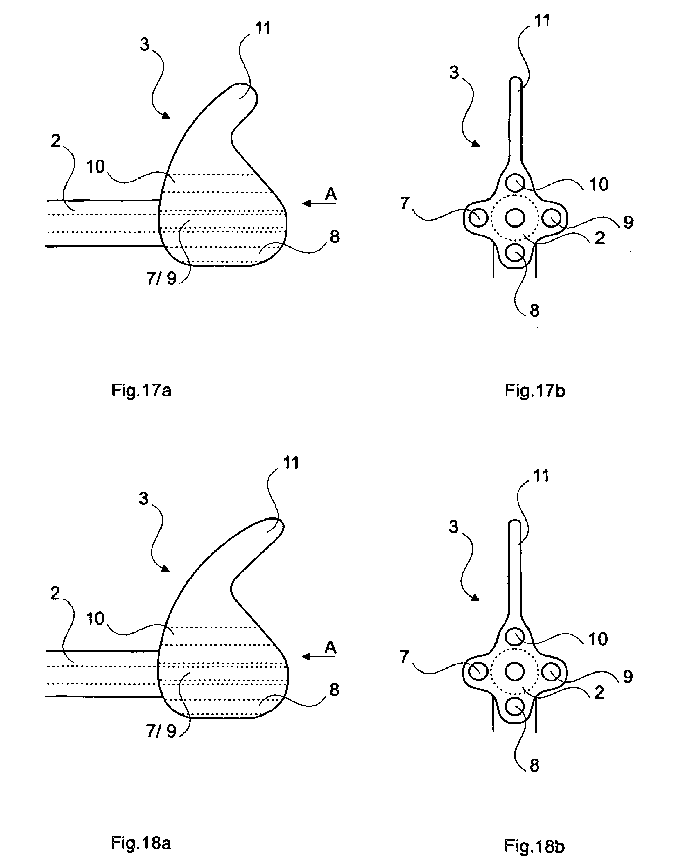

Fig.17a und Fig.17b stellen den Positionierhaken 3 des Positionier- und Einführwerkzeuges 1, der keine Führungsbohrung aufweist dar, mit verschiedenen Korrekturbohrungen, insbesondere eine anterior/posterior Korrekturbohrung 7, eine mediale Korrekturbohrung 8, eine posterior/anterior Korrekturbohrung 9 und eine laterale Korrekturbohrung 10. Diese Variante unterscheidet sich im Vergleich zu den in Fig. 15a und Fig.15b, Fig.16a und Fig.16b dargestellten Varianten im wesentlichen durch eine geringere Querschnittsfläche. Die Korrekturbohrungen werden von der Außenkontur umschlossen.

Fig.18a und Fig.18b stellen den Positionierhaken 3 des Positionier- und Einführwerkzeuges 1, der keine Führungsbohrung aufweist dar, mit verschiedenen Korrekturbohrungen, insbesondere eine anterior/posterior Korrekturbohrung 7, eine mediale Korrekturbohrung 8, eine posterior/anterior Korrekturbohrung 9 und eine laterale Korrekturbohrung 10. Im Vergleich zu der in Fig.17a und Fig.17b dargestellten Variante läuft die Nase etwas spitzer aus und ist länger. Diese Variante unterscheidet sich im Vergleich zu den in Fig.15a und Fig.15b, Fig.16a und Fig.16b dargestellten Varianten im wesentlichen durch eine geringere Querschnittsfläche. Die Korrekturbohrungen werden von der Außenkontur umschlossen.

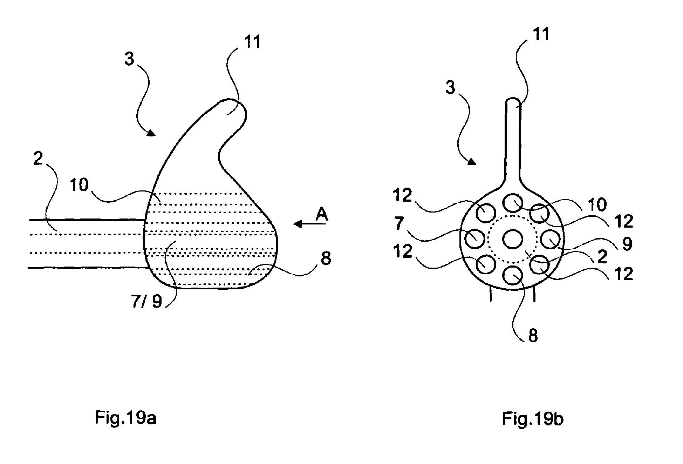

Fig.19a und Fig.19b stellen den Positionierhaken 3 des Positionier- und Einführwerkzeuges 1, der keine Führungsbohrung aufweist dar, mit verschiedenen Korrekturbohrungen, insbesondere eine anterior/posterior Korrekturbohrung 7, eine mediale Korrekturbohrung 8, eine posterior/anterior Korrekturbohrung 9, eine laterale Korrekturbohrung 10 und vier Zusatz-Korrekturbohrungen.

### Bezugszeichenliste

- 1 -: Positionier- und Einführwerkzeug
- 2 -: Führungsrohr
- 3 -: Positionierhaken
- 4 -: Haltearm
- 5 -: Handgriff
- 6 -: Führungsbohrung
- 7 -: anterior/posterior Korrekturbohrung
- 8 -: mediale Korrekturbohrung
- 9 -: posterior/anterior Korrekturbohrung
- 10 -: laterale Korrekturbohrung
- 11 -: Nase
- 12 -: Zusatz-Korrekturbohrung
- 20 -: Öffnungsbohrer bzw. -fräser
- 30 -: T-Griff
- 31 -: Bohrfutter
- 32 -: Hebel
- 40 -: Femur
- 41 -: Trochanter
- 42 -: Winkel
- 43 -: Öffnung
- 44 -: Femurachse
- 45 -: medialer Muskel
- 50 -: Führungsdraht
- 50a -: Referenz-Führungsdraht
- 50b -: Öffnungs-Führungsdraht
- 50b1 -: weiterer Öffnungs-Führungsdraht

## Patentansprüche

1. Gebogenes Positionier- und Einführwerkzeug für das Einbringen eines Führungsdrahtes (50) in den Femur (40) mit einem gebogenen Führungsrohr (2) mit einem distalen Ende zum Ansetzen am Trochanter (41) und/oder den medialen Muskel (45) und einem proximalen Ende für das Einschieben des Führungsdrahtes (50), mit einem Haltearm (4) für die Verbindung zwischen dem Führungsrohr (2) und einem Handgriff (5), **dadurch gekennzeichnet, dass** am distalen Ende des Führungsrohres (2) ein Positionierhaken (3) angeordnet ist, der mit wenigstens einer Korrekturbohrung (7, 8, 9, 10, 12) ausgerüstet ist, für die Aufnahme des einen oder eines anderen Führungsdrahtes (50).

2. Werkzeug nach Anspruch 1, **dadurch gekennzeichnet, dass** der Positionierhaken (3) wenigstens vier, vorzugsweise acht Korrekturbohrungen (7, 8, 9, 10, 12) aufweist, die insbesondere an einem konzentrischen Kreis rund um das Führungsrohr (2) ausgerüstet ist.

3. Werkzeug nach einem der vorhergehenden Ansprüche, **dadurch gekennzeichnet, dass** der Positionierhaken (3) mit wenigstens einer Führungsbohrung (6) ausgerüstet ist, für die Aufnahme des einen oder eines anderen Führungsdrahtes (50).

4. Werkzeug nach einem der vorhergehenden Ansprüche, **dadurch gekennzeichnet, dass** die Ausgestaltung des Positionierhakens (3) nasenförmig und derart gewählt ist, dass er beim Aufsetzen auf den Trochanter 41 in der optimalen Position für das Einbringen eines Öffnungs-Führungsdrahtes (50b) in den Femur (40) zu liegen kommt.

5. Werkzeug nach Anspruch 3 oder nach Anspruch 4 in Abhängigkeit von Anspruch 3 **dadurch gekennzeichnet, dass** das Führungsrohr (2) wie auch die Führungsbohrung (6) so ausgebildet sind, dass sie sowohl einen Referenz-Führungsdraht (50a), als auch einen Öffnungs-Führungsdraht (50b) aufnehmen können.

6. Werkzeug nach einem der vorhergehenden Ansprüche, **dadurch gekennzeichnet, dass** der Positionierhaken über wenigstens eine anterior/posteriore (7) und/oder eine mediale (8) und/oder eine posterior/anteriore (9) und/oder eine laterale Korrekturbohrung (10) verfügt, um zweite oder weitere Offnungs-Führungsdrähte (50b1) in bestimmtem Abstand zum ersten Offnungs-Führungsdraht (50b) in den Femur (40) einschlagen oder bohren zu können.

7. Werkzeug nach einem der vorhergehenden Ansprüche, **dadurch gekennzeichnet, dass** der Handgriff (5) in Einführposition Im wesentlichen parallel zur frontalen Femurachse (44) ausgerichtet ist.

8. Werkzeug nach einem der vorhergehenden Ansprüche, **dadurch gekennzeichnet, dass** der Positionierhaken (3) mit dem Führungsrohr (2) fest verbunden ist.

9. Werkzeug nach Anspruch 3 oder nach einem der Ansprüche 4 - 8 in Abhängigkeit von Anspruch 3 **dadurch gekennzeichnet, dass** die Fohrungsbohrung (6) in einem Winkel (42) von 6°-30°, vorzugsweise 7°-20°, insbesondere ca. 8° tangential zur Achse des Führungsrohres (2) im Bereich seines distalen Endes erstreckt ist.

10. Werkzeug nach einem der vorhergehenden Ansprüche, **dadurch gekennzeichnet, dass** die Korrekturbohrungen (7) in einem Winkel zur Achse des Führungsrohrs (2) erstreckt sind.

11. Werkzeug nach einem der vorhergehenden Ansprüche, **dadurch gekennzeichnet, dass** der Positionierhaken (3) eine besondere rutschsichere Ausbildung zum Ansatz auf dem Trochanter (41) aufweist.

12. Werkzeug nach einem der vorhergehenden Ansprüche, **dadurch gekennzeichnet, dass** der Positionierhaken (3) eine verrundet und schmal ausgestaltete Nase (11) aufweist, die den Positionierhaken weichteilschonend im Muskelgewebe hält.

13. Set aus Werkzeugen nach einem der Ansprüche 1-12, **dadurch gekennzeichnet, dass** mehrere unterschiedlich beschaffene Positionierhaken (3) vorgesehen sind, aus denen je nach Trochanter- und Weichteilbeschaffenheit ein angepasstes Werkzeug wählbar ist.

14. Werkzeug nach einem der Ansprüche 1 bis 12, **dadurch gekennzeichnet, dass** das Führungsrohr in einem Radius von ca. 300 bis ca. 800 mm, vorzugsweise 500 bis 700 mm, insbesondere ca. 600 mm gebogen ist.

## Claims

1. Curved positioning and insertion instrument for inserting a guide wire (50) into the femur (40), comprising a curved guide tube (2) having a distal end for placing on the trochanter (41) and/or the medial muscle (45) and a proximal end for insertion of the guide wire (50), comprising a retaining arm (4) for the connection between the guide tube (2) and a handle (5), **characterized in that** a positioning hook (3) is arranged at the distal end of the guide tube (2) and is equipped with at least one correction bore (7,8,9,10,12)for receiving the one guide wire (50) or another guide wire (50).

2. Instrument according to Claim 1, **characterized in that** the positioning hook (3) has at least four, preferably eight, correction bores (7,8,9,10,12) which are provided in particular on a concentric circle around the guide tube (2).

3. Instrument according to either of the preceding Claims, **characterized in that** the positioning hook (3) is equipped with at least one guide bore (6) for receiving the one guide wire (50) or another guide wire (50).

4. Instrument according to any of the preceding Claims, **characterized in that** the design of the positioning hook (3) is nose-shaped and is chosen so that, when placed on the trochanter (41), it comes to rest in the optimum position for the insertion of an opening guide wire (50b) into the femur (40).

5. Instrument according to any of Claims 3 - 4, **characterized in that** the guide tube (2) as well as the guide bore (6) are formed in such a way that they can receive both a reference guide wire (50a) and an opening guide wire (50b).

6. Instrument according to any of the preceding Claims, **characterized in that** the positioning hook has at least one anterior/posterior (7) and/or one medial (8) and/or one posterior/anterior (9) and/or one lateral correction bore (10) in order to be able to tap or drill second or further opening guide wires (50b1) a certain distance away from the first opening guide wire (50b) into the femur (40).

7. Instrument according to any of the preceding Claims, **characterized in that** the handle (5) is aligned in the insertion position substantially parallel to the frontal femur axis (44).

8. Instrument according to any of the preceding Claims, **characterized in that** the positioning hook (3) is firmly connected to the guide tube (2).

9. Instrument according to any of Claims 3 to 8, **characterized in that** the guide bore (6) extends at an angle (42) of 6°-30°, preferably 7°-20°, in particular about 8°, tangentially to the axis of the guide tube (2) in the region of its distal end.

10. Instrument according to any of the preceding Claims, **characterized in that** the correction bores (7) extend at an angle to the axis of the guide tube (2).

11. Instrument according to any of the preceding Claims, **characterized in that** the positioning hook (3) has a particular non slip design for placing on the trochanter (41).

12. Instrument according to any of the preceding Claims, **characterized in that** the positioning hook (3) has a rounded and narrow nose (11) which holds the positioning hook in the muscle tissue with protection of soft tissue.

13. Set of instruments according to any of Claims 1-12, **characterized in that** a plurality of positioning hooks (3) having different characteristics are provided, from which a suitable instrument can be chosen depending on the characteristics of trochanter and soft tissue.

14. Instrument according to any of Claims 1 - 12, **characterized in that** the guide tube is curved with a radius of about 300 to 800mm, preferably 500 to 700mm, in particular about 600mm.

## Revendications

1. Outil de positionnement et d'insertion incurvé destiné à introduire un fil-guide (50) dans le fémur (40) avec un tube de guidage (2) incurvé, avec une extrémité distale devant être fixée sur le trochanter (41) et/ou sur le muscle médian (45) et une extrémité proximale destinée à introduire le fil-guide (50), comportant un bras de maintien (4) destiné à établir la liaison entre le tube de guidage (2) et une poignée (5), **caractérisé en ce que**, au niveau de l'extrémité distale du tube de guidage (2) est disposé un crochet de positionnement (3), qui est doté d'au moins un alésage correcteur (7, 8, 9, 10, 12), destiné à recevoir l'un ou l'autre fil-guide (50).

2. Outil selon la revendication 1, **caractérisé en ce que** le crochet de positionnement (3) présente au moins quatre, de préférence huit alésages correcteurs (7, 8, 9, 10, 12), qui sont en particulier disposés selon un cercle concentrique tout autour du tube de guidage (2).

3. Outil selon l'une quelconque des revendications précédentes, **caractérisé en ce que** le crochet de positionnement (3) est doté d'au moins un alésage de guidage (6), destiné à recevoir l'un ou l'autre fil-guide (50).

4. Outil selon l'une quelconque des revendications précédentes, **caractérisé en ce que** la configuration du crochet de positionnement (3) est en forme de nez et est choisie de telle sorte que, lorsqu'on le pose sur le trochanter 41 dans la position optimale pour introduire un fil-guide d'ouverture (50b), il vient se placer dans le fémur (40).

5. Outil selon l'une quelconque des revendications 3 - 4, **caractérisé en ce que** le tube de guidage (2) ainsi que l'alésage de guidage (6) sont configurés de telle sorte qu'ils peuvent recevoir aussi bien un fil-guide de référence (50a) qu'un fil-guide d'ouverture (50b).

6. Outil selon l'une quelconque des revendications précédentes, **caractérisé en ce que** le crochet de positionnement dispose d'au moins un alésage correcteur antérieur/postérieur (7) et/ou médian (8) et/ou postérieur/antérieur (9) et/ou latéral (10), afin de pouvoir enfoncer ou introduire les deuxièmes ou autres fils-guides d'ouverture (50b1) à une distance déterminée du premier fil-guide d'ouverture (50b) dans le fémur (40).

7. Outil selon l'une quelconque des revendications précédentes, **caractérisé en ce que** la poignée (5), dans la position d'introduction, est orientée sensiblement parallèlement à l'axe du fémur frontal (44).

8. Outil selon l'une quelconque des revendications précédentes, **caractérisé en ce que** le crochet de positionnement (3) est relié fermement au tube de guidage (2).

9. Outil selon l'une quelconque des revendications 3 - 8, **caractérisé en ce que** l'alésage de guidage (6) s'étend selon un angle (42) compris entre 6° et 30°, de préférence entre 7° et 20°, en particulier égal à environ 8° de manière tangentielle par rapport à l'axe du tube de guidage (2) dans la zone de son extrémité distale.

10. Outil selon l'une quelconque des revendications précédentes, **caractérisé en ce que** les alésages correcteurs (7) s'étendent selon un certain angle par rapport à l'axe du tube de guidage (2).

11. Outil selon l'une quelconque des revendications précédentes, **caractérisé en ce que** le crochet de positionnement (3) présente une configuration résistant au glissement spécifique afin de se fixer sur le trochanter (41).

12. Outil selon l'une quelconque des revendications précédentes, **caractérisé en ce que** le crochet de positionnement (3) présente un taquet (11) configuré de manière arrondie et étroite, qui maintient le crochet de positionnement dans le tissu musculaire en épargnant la partie molle.

13. Ensemble d'outils selon l'une quelconque des revendications 1 à 12, **caractérisé en ce que** plusieurs crochets de positionnement (3) présentant une texture différente sont prévus, parmi lesquels on peut choisir un outil adapté en fonction de la texture de la partie molle et du trochanter.

14. Outil selon l'une quelconque des revendications 1 à 12, **caractérisé en ce que** le tube de guidage est incurvé sur un rayon compris entre environ 300 et environ 800 mm, de préférence entre 500 et 700 mm, en particulier égal à environ 600 mm.
